# EUROPEAN PATENT SPECIFICATION

(11) **EP 1 487 847 B1**
(45) Date of publication and mention of the grant of the patent: **16.08.2006**
(21) Application number: 02717016.6
(22) Date of filing: 25.03.2002
(51) Int. Cl.: C07H 15/256, C07H 1/08

(54) **A SIMPLE PROCESS FOR OBTAINING BETA-AESCIN FROM INDIAN HORSE CHESTNUT (AESCULUS INDICA)**
EIN EINFACHES VERFAHREN ZUR GEWINNUNG VON BETA-AESCIN AUS DER INDISCHEN ROSSKASTANIE (AESCULUS INDICA)
PROCESSUS SIMPLE D'OBTENTION DE BETA-AESCINE A PARTIR DE MARRONNIER DE L'HIMALAYA (AESCULUS INDICA)

(43) Date of publication of application: 22.12.2004
(73) Proprietor: COUNCIL OF SCIENTIFIC AND INDUSTRIAL RESEARCH, New Delhi 110 001 (IN)
(72) Inventor: SINGH, Bikram, 176 061 Himachal Pradesh (IN)
(74) Representative: Appelt, Christian W.
(86) International application number: PCT/IB2002/001208
(87) International publication number: WO 2003/080636

(56) References cited:
- EP-A- 0 298 148
- DE-A- 2 530 941
- GB-A- 1 539 127
- US-A- 3 163 636
- US-A- 3 766 166
- SRIJAYANTA, S. ET AL.: "A COMPARATIVE STUDY OF THE CONSTITUENTS OF AESCULUS HIPPOCASTANUM AND AESCULUS INDICA" J. MED. FOOD, vol. 2, no. 2, 1999, pages 45-50, XP008007007 cited in the application

## Description

### Technical Field

The present invention relates to development of a simplified process for obtaining β-aescin from Indian horse chestnut *(Aesculus indica)*

Particularly this invention relates to separation of β-aescin from Indian horse chestnut by aqueous alcohol extraction, solvent - solvent partition, treatment with base followed by passing the organic layer through acidic alumina. More particularly this invention relates to purification of β-aescin from Indian horse chestnut (*A. indica*) in 2.0-3.0% yield of desired specifications.

### Background art

*Aesculus indica* Hook. (Hippocastanaceae) (Indian horse chestnut) is known as Bankhor in Hindi. A large, deciduous tree, with a short, straight, cylindrical bole and spreading crown, 30m in height and 7.5m in girth in favourable localities, distributed in the Himalayas from Kashmir to west Nepal, at altitudes of 900-3,600m. The tree is very handsome and generally in rich, moist, shady ravines of Kashmir, Himachal Pradesh and Uttar Pradesh up to Nepal. It is often planted as ornamental on the hills. The tree sheds leaves from October to early December and the new leaves appear in April, and flowers during April-June when it is extremely attractive. The shoots and leaves are used as fodder and cattle eat the fruits. In times of scarcity, the hill tribes also eat them, after steeping in water to remove the bitter components, or sometimes the powdered seeds are mixed with wheat flour. The crushed fruit is used for washing clothes. Analysis of the seeds gave: moisture, 3.5; reducing sugars (as dextrose), .83; sucrose, 9.07; starch, 38.3; water sol, 36.4; and Crushed seeds, if fed to cattle, are reported to improve the quality and quantity of milk. Seeds are also given to horses for colic. The seeds contain oil, which is used in rheumatism; it is also applied to wounds. The wood is creamy white or pale pinkish white when freshly cut, becomes pinkish brown on exposure, with light brown lines; the heartwood is not distinct. The timber is dull, with a smooth feel, and without any characteristic odour. It is light to moderately heavy (Sp. Gr. 0.43-0.57; wt, 479-560 kg/cum), soft, straight-or narrowly and shallowly interlocked-grained and sometimes waxy-grained. It is easy to saw, finishes to a smooth surface and takes good polish. The timber seasons well without much degrade.

The timber is largely used for building, packing cases, water-troughs, planking, cooperage, platters, tea-boxes, boat-fittings, cabinet-making and turnery articles; it is also suitable for mathematical instruments, shoe-heels and match-splints. The wood is also used for bobbins. The hardwood, after suitable dyeing and waxing, has been found to be suitable for high quality pencils. Selected portions are useful for high paneling in railway carriages, and for sports-goods. The bark is made into a paste for application on painful, dislocated joints. Extract of the stem bark is reported to possess fungicidal properties. The roots are used as a cure for leucorrhoea. (Annon., 1985, The Wealth of India, Raw Material, CSIR, New Delhi, 1,95).

*A. indica* has been investigated for chemical and biological studies by different groups. Starch (25%) from Indian horse chestnut on dry basis was reported (Uppal, I. S., 1952, Starch from Indian horse chestnuts and its chemical examination, J. Ind. Chem. Soc., 15, 178-80). Two flavonoids glycosides namely, rutin and astragalin from the chloroform extract of the stems, a flavonoids glycoside quercitrin, β-sitosterol and a triterpenoid saponin, aescin from the leaves (Ikram, M., Khan M. I., Kawano, N., 1978, Chemical investigation of *Aesculus indica*, Planta Medica, 34, 337-338; Ikram, M., Khan M. I., 1978, Chemical investigation of *Aesculus indica* Part-II, Fitoterapia, 49, 247-248). Aliphatic compounds namely *n*-hentriacontane, *n*-hentriacontanol and palmitone along with β-sitosterol from various extractives of leaves and a coumarin glycoside (aesculin) and quercetin L-rhamnoside (Mukherjee, K. S., Bhattacharya, M. K., Ghosh, P. K., 1983, Phytochemical investigation of *Aesculus indica* Linn. and *Fragaria indica* Andr. J. Indian Chem. Soc., 60, 507-508). Different triterpene glycosides (3-5) from Indian horse chestnuts were also reported by other workers (Singh, B., Agrawal, P. K., Thakur, R. S., 1986, Aesculuside-A, a new triterpene glycoside from *Aesculus indica,* Planta Medica, 52, 409-410; Sati, O. P., Rana, U., 1987, A new molluscicidal triterpenic glycoside from *Aesculus indica,* Int. J. Crude Drug Res., 25, 158-160; Singh, B., Agrawal, P. K., Thakur, R. S., 1986, Aesculuside-B, a new triterpene glycoside from *Aesculus indica*, J. Nat. Prod., 50, 781-783). In a comparative study of phytochemicals in *Aesculus indica* and *A. hippocastanum* seeds, husk & leaves depicted the differences between the species. Aesculin and its aglycone, aesculetin were not detected in leaves and husk of two species. Seeds and leaves had similar components whereas they varied in husk. The content of aescin found to be 13.4% (w/w) in *A. indica* whereas *A. hippocastanum* contained 9.5%. Lauric, palmitic, myristic stearic, arachidic and oleic acid were detected by capillary GC. (Srijayanta S.; Raman A.; Goodwin B.L. A comparative study of the constituents of *Aesculus hippocastanum* and *A. indica*, Jour. Med. Food, Vol. 2(2), 1999, 45-50). Earlier reports, indicated the presence of aescin in *A. hippocastanum* as more than 13% (Shibata, S., 1977, New natural products and plant drugs with pharmacological, biological and therapeutical activity (Wagner H., Wulff, P. (ed) p 177, Springer, Berlin), whereas it was reported to be 3-6% in *A. hippocastanum* (Hostettmann, K. Marston, 1995, A., Saponins, in Chemistry and Pharmacology of Natural Products, Cambridge University Press)

The seeds of *Aesculus* species are known as horse chestnuts and used as food, feed and fodder, for the production of alcohol. They possess therapeutic importance in human and veterinary medicines, e.g. in the treatment of fevers, for the use in curing piles, obstinate constipation, of mammary in duration and cancer. The meal of horse chestnuts is employed to cleanse oily skin. Due to their saponin contents, the seeds possess toxic properties. Alcoholic extracts of seeds of *A. hippocastanum* show hoemolytic, antiedema and other pharmacological properties. Extracts have been also applied in cosmetics whereas the oil extracted from *A. indica* seeds exhibits a significant anti-inflammatory activity in carrageenin-induced edema in rats. Sometimes, addition of other plant extracts or chemical compounds enhances their activity or caused a change in biological effects (Hostettmann, K. Marston, 1995, A., Saponins, in Chemistry and Pharmacology of Natural Products, Cambridge Univerisity Press; Kirtikar, K.R., Basu, B.D., 1935, Glossary of Indian Medicinal Plants L.M. Basu, Allahabad, India Vol. I, p. 626; Singh, B. Agrawal, P. K., 1996, Chemistry and Biological Activity of Aescin, in Supplements to Cultivation and Utilization of Medicinal Plants, (eds.) S.S. Handa and M.K. Kaul, PP 457-476 (RRL Jammu, CSIR).

Aescin is known for its anti-inflammatory and anti-endemic activities and is used in tropical applications of ointment and oral preparations for the treatment of peripheral vascular diseases (Bruneton, J., 1995, Pharmacognosy, Phytochemistry and medicinal plants, Paris, France, Lavoisier). Hypoglycemic and anti-inflammatory activities of different derivative of aescin isolated from horse chestnut seeds was demonstrated (Yoshikawa, M., Murakami, T., Yamahara, J., 1998, Bioactive saponins and glycosides. XII horse chestnut (2), structure of escin IIIb, IV and VI and isoescins la, Ib and V, acylated polyhydroxy oleanane triterpene oligoglycosides, from the seeds of horse chestnut tree *(Aesculus hippocastanum* L., Hippocastanaceae), Chemical and pharmaceutical bulletin 46, 1764-1769)

Horse chestnut extract is reported to have beneficial effects on venous insufficiency and associated conditions. Aescin exhibited potent anti-inflammatory active, also reduces capillary fragility and therefore, helped to prevent leakage of fluid into surrounding tissues, which can cause swelling. Horse chestnut extract was also found to have powerful anti-oxidant than Vitamin -E. Among 65 plant extracts tested, horse chestnut extract was shown to have one of the highest 'active-oxygen' scavenging abilities. It exhibited potent cell protective effect. These activities are linked with anti-aging properties of anti-oxidants. The extract is also rich in a number of flavonoids, such as derivatives of quercetin and kaempferol. Flavonoids also have protective effect on blood vessels, and are well known, powerful antioxidants (Wilkinson, J.A., Brown, A. M. G., 1999, Horse chestnut-*Aesculus hippocastanum*. Potential application in cosmetic skin-care products, International Journal of Cosmetic Science, Vol. 21(6), 437-447. Pharmaceutical preparations containing aescin have been prepared for treatment of various types of edema and venous circulatory system disorders. The dry seeds extract processed to form pellets with dextrin. The final products can be best provided as hard gelatin capsule or matrix tablets containing extract (Tobin, J., Heese, G. U., 1999, Production of pharmaceutical formulation containing aescin for treatment of edema & venous disorder EP, 900563A1). A cosmetic and paramedical composition containing aqueous-ethanol extracts of marigold, horse chestnut, licorice, aqueous extracts of silverweed, walnut tree leaves, lavender resinoid and Roman chamomile with ethanol-water carrier in fixed proportions. The above preparation serves as a base material for excellent cosmetic composition, curative cosmetic & curative products. (Hangay, G., Kelen, A., Ranky, S. K., Gulyas, A., Simonovits, E., Vinezenee, K. J., Szepesi, G., Kesera, P., Selmeczi, A., Putz, P., 1992, Cosmetic and paramedical composition containing plant extracts, US 5080901). The use of aescin complexes with β-sitosterol or cholesterol and phospholipids as pharmaceutical composition like anti-inflammatory acid vasotonic agent is described (Bombardelli E., Patri G., Pozzi R., Complexes of aescin, β-sitosterol or cholesterol and phospholipids and pharmaceutical compositions containing them, US 5118671, 1992).

In another, which relates to a medicament containing a standard dry extract of horse chestnut seeds active against various types of edema and diseases of venous circulatory system and a process for manufacturing of this medicament. In this process, the powder is extracted with alcoholic and water to obtain alcohol tincture. The tincture is mixed with dextrin and spray dried for further formation of pellets with polyvinyl pyrrolidone, talcum. This is further moistened with alcohol and palletizing is done with the help of mixer/granulator apparatus or marumerizer, spheronizer or other palletizing material. The above preparation containing standard extract for treatment of venous circulatory disorders venous insufficiency inflammations, edema including brain edema or swelling (Toblin, J., Heese, G. U., 2000, Production of pharmaceutical formulations for treatment of edema and venous disorders, US 6077534).

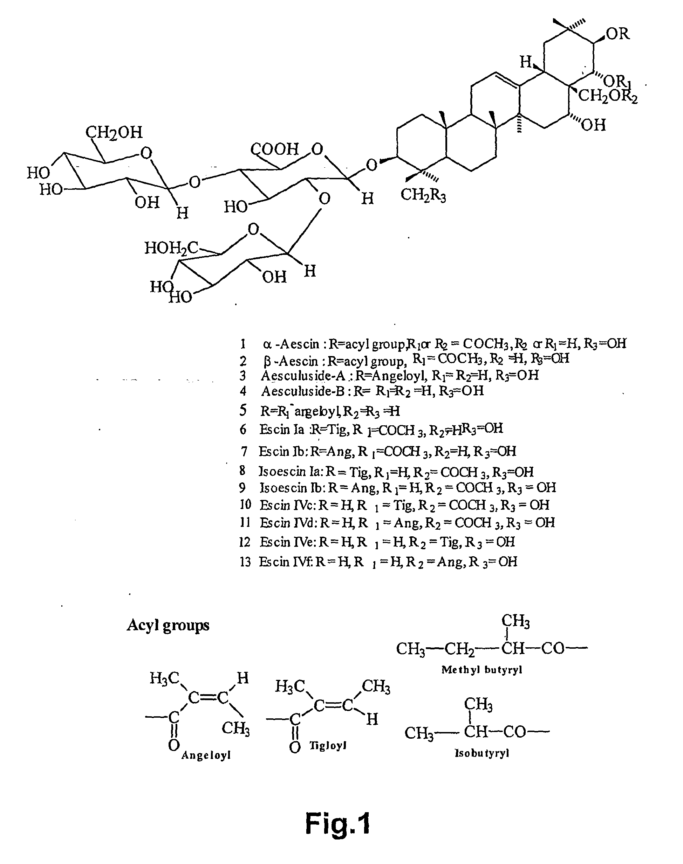

Aescin also known as Escin, is a mixture of triterpene saponins and is the main bioactive constituent of horse chestnut seeds, twigs, sprouts and leaves (Mete, K. O., Kara, M., Kara, S., 1994, Quantitative determination of escin. A comparative study of HPLC and TLC-densitometry, Fitoterapia, 65, 439-443). A saponified fraction of seeds showed the presence of various fatty acids including arachidic, stearic, oleic and Lauric acids (Srijayanta S.; Raman A.; Goodwin B.L.) Aescin exists in two forms as β-aescin and α-aescin. β-Aescin, which is, derived from protoaescigenun and barringtogenol, occurring in the ratio of 8:2 (Bombardelli, E., Morazzoni, P., Griffini, A., 1996, *Aesculus hippocastanum* L., Fitoterapia, 67, 483-511). Seed oil contains 65-70% oleic acid. Other constituents include phenolic acids, coumarin derivative Aesculin, hydrocarbons such as squalene and nonacosane and cyclitols (Stankovic, S. K., Bastic, M. B., Jovanovic, J. A., 1987, Ontogenetic distribution of hydrocarbons in horse chestnut seed *(Aesculus hippocastanum* L.), Herba Polonica, 33, 3-8). Aesculin extracted from the bark of horse chestnut is used in preparations for the treatment of peripheral vascular diseases, hemorrhoids and cosmetics designed to ameliorate aging skin (Bombardelli, E., Morazzoni, P., Griffini, A., 1996, *Aesculus hippocastanum* L., Fitoterapia, 67, 483-511). Overall, seeds contain 3% water, 3% ash, 11 % crude protein, 5% oil and 74% carbohydrates (Duke, J. A., 1985, CRC Handbook of medicinal herbs, Boca Raton, FL, CRC Press). Recently eight bioactive saponins including four new compounds have been reported from *Aesculus chinensis.* The major being escin la **(6)**, escin Ib **(7)**, isoescin la **(8)** and isoescin Ib **(9)** and the new compounds escin IVc (22α-tigloyl-28-acetyl protoaescigenin-3β-O-[β-D-glucopyranosyl (1-2)] [β-D-glucoppyranosyl (1-4)]-β-D-glucopyranosiduronic acid) **(10)**, escin IVd (2α-Angeloyl-28-acetlyprotoaescigenin-3β-O-[β-D-glucopyranosyl (1-2)] [β-D-glucopyranosyl (1-4)]-β-D-glucopyranosiduronic acid) **(11)**, escin IVe (28-tigloylproaescigenin-3β-O-[β-D-glucopyranosyl (1-2)] [β-D-glucopyranosyl (1-4)]-β -D-gluco-pyranosiduronic acid) **(12)**, escin IVf (28-angeloylprotoaescigenin-3β-O-[β-D-glucopyranosyl (1-2)] [β-D-glucopyranosyl (1-4)]-β-D-gluco-pyranosiduronic acid) **(13)**. These compounds were reported to have inhibitory activity against HIV-1 protease. Some of the major compounds present in aescin are listed in fig. 1 (Yang X-W.; Zhao J.; Cin Y-X.; Lin X-H.; Mac-M.; Hattori M.; Zhang L-H.; 1999, Anti-HIV-1 Protease Triterpenoid saponins from the seeds of *Aesculus chinensis,* Jour. Nat. Prod., 62(11) 1510-1513. An Anti-inflammatory agent comprising of aescin Ib & IIb with angeloyl groups which is a saponin ingredients of *Aesculus hippocastanum* have been prepared. The purification of aescin Ib & IIb was carried out from butanol fraction of methanol extract after column chromatography followed by high performance liquid chromatography. (Yamahara, J., Araki, N., Otsuji, K., 1998, Antiinflammatory Agent, JP 10017591A2).

The aescin was purified from horse chestnut seed extract by absorbing the aqueous solution of seed on synthetic polymer (polar and medium polar). The resin containing absorbed aescin is washed thoroughly with water and made to contact with a hydrophilic organic solvent or its mixture with water to obtain the aescin fraction separated from impurities. Drawback of the invention was that synthetic resin in the form of polymers were used for purification of aescin thus enhancement of the production costs. (Ogawa, S., Maruzeu, K. K. K., 1987, Purification of aescin, JP 62081325A2). A process was reported for production of aescin rich concentrate from horse chestnut seed by defatting the seeds and extracting with weak acids like acetic/formic acid followed by making the seeds free from fats with low molecular weight alcohol or acetone. The extract was further dried to obtain aescin rich fraction. In this process, the drawback was that the powdered seeds were treated first with hexane to defatt the material and then with weak acid followed by drying. The dried powder was then extracted with low molecular weight alcohol to give aescin rich concentrate (Menssen, H. G., Geyen, M., 1971, Process for the production of an aescin rich concentrate of active material from horse chestnut seeds, US 3609137).

Alternatively, aescin was extracted from horse chestnut by formation of complexes. Thus, defatted horse chestnuts were treated with propylene glycol, 1,3, bulylene glycol or glycerol with water ethanol or water isopropanol solution to yield aescin 2.5% which was considered to be a more stable component. The drawback of the process was that the powdered horse chestnut were first defatted followed by the formation of complex with propylene glycol, 1,3, bulylene glycol or glycerol and then extraction with aqueous organic solvent, liberation of aescin from complex followed crystallization to yield 2.5% aescin. This process increased the number of purification steps involved, thereby increasing the production cost. (Dzienget, K., 1964, Stable saponin containing extract from horse chestnut Ger. 1,182,385). In an another method, the aescin in the form of cholesterol was extracted by treating the seeds with 10% aqueous alcohol with cholesterol in ether and stirring for 1 hr. at 90°C. Ether was evaporated and residue centrifuged and washed with water till colorless. The residue was treated with methanol and the material filtered off. The solution was decolorized with active carbon. The methanol removed in vacuum and residue dried to give aescin. The major drawback in this process was that the powder was subjected to a number of steps by the formation of complex with cholesterol, defatting extraction, liberation of complex, centrifugation of extracted material, decolourization of extract etc. Thus, the production cost was increased. (Wagner, J., bosse, J" 1964, Genuine aescin from horse chestnut extract, US 3163636).

Aescin from *A. hippocastanum* was also separated in crystalline form by extracting the ground seeds with water-miscible and passing the extract over cation exchange resin. The eluant was precipitated to get crude aescin by redissolving in methanol and precipitating with water. The drawback of this process was that the extract was made to pass through cation exchange resin, which is a very costly affair. Thus, cost of production is raised. (Rolf, M., 1977, *Aesculus hippocastanum* saponin and salts, Ger. 2530941). In another process for isolation of β-escin, the dried ground fruits of horse chestnut were treated with C₄₋₅ aliphatic alcohol and water saturated with alcohol. The aescin was extracted from organic phase after effecting change in polarity by addition of a C₄₋₅ aliphatic ester preferably ethyl acetate with bicarbonate at pH 5.0-5.5. The ratio of α:β varied from 10:90 to 20:80 depending on the source and agricultural conditions. Drawback of this process was that the number of extracting steps were increased thus making the process more lengthy and cost ineffective. (Rudolf, R., Stanstav, J., 1978, Isolation of escin from horse chestnuts Span. 455,693). In yet another method, the aescin was extracted from ground horse chestnuts with mixture of water saturated alcohol and alcohol saturated with water. The organic phase was purified by polarity change with ethyl acetate, further treatment with sodium bicarbonate at pH 5.0-5.5 which gave sodium salt of escin. This was decolorized with active carbon and followed by passing through cation exchange resin to yield aescin in 2.2% yield with 220-30°C mp. Drawback of this process here again was that the number of extracting steps were increased by polarity change with ethyl acetate, treatment with sodium bicarbonate, passing through cation exchange resin to yield aescin in 2.2% yield thus making the process more lengthy, tedious and cost ineffective. (LEK, Tovarm, 1976, isolation of aescin from horse chestnut, Ger. 233204). In an another process, the horse chestnut were treated with an aqueous solution at pH<3 with hydrochloric acid followed by repeated extraction with aliphatic alcohol C₃₋₆ and chlorinated C₁₋₂ hydrocarbon to yield pure aescin. Drawback of this process was that acid and number of organic solvents were used to give aescin in low yield (less than 3%) (Knoll, A.G., 1967, Quantitative recovery of pure escin from horse chestnut extract, Fr. 1,587,868).

In all the above mentioned processes for extraction and purification of aescin from horse chestnut *(Aesculus* species) either the methodology used is lengthy and tedious whereby, increasing the number of steps involved or other components are used for achieving the extraction and purification like complexes formation, use of resins (cation/anion), active carbon, maintenance of pH by using acid/bases and polarity change are involved and production cost is increased. Whereas, we describe a simplified, easy and convenient process for extraction and purification of aescin from Indian horse chestnut *(Aesculus indica*)

### Objects of the invention

The main object of the present investigation is to develop of a simple process for purification of aescin from Indian horse chestnut *(Aesculus indica)*

Another object of the present invention is to develop easy and convenient commercial extraction process for aescin, a component of pharmaceutical

importance from Indian horse chestnut *(Aesculus indica* seeds) growing wild in high hills of Himalayas and goes unutilized every year in hundreds of tons.

Still another object of the present investigation is to provide simple and convenient process for purification of aescin from Indian horse chestnut in 2.0-3.0% yield of desired specifications.

### Summary of the Invention

Accordingly, the present invention provides a simple process for preparing β-aescin from Indian horse chestnut, which overcome the drawbacks of the prior art.

The purity of the product β-aescin is more than 90 % and comparable to the standard β-aescin available commercially.

### Detailed Description of the Invention

Accordingly, the present provides a process for obtaining β-aescin from Indian horse chestnut *(Aesculus indica)* said process-comprising steps of:
a) extracting powdered Indian horse chestnut seeds with mixture of water and low molecular alcohol, said low molecular alcohol being selected for the group consisting of methanol and ethanol,
b) filtering the solution of step (a) to provide aqueous alkanol extract,
c) removing the alkanol from step (b) to provide essentially an aqueous extract,
d) partitioning the aqueous extract of step (c) with water immiscible lower alkanol saturated with water, wherein said water immiscible alkanol is selected from a group consisting of n-butanol and n-propanol or their mixture thereof, preferably n-butanol,
e) separating the organic layer and aqueous layer of step (d),
f) treating the organic layer of step (e) with 0.5 to 1.0% aqueous alkali hydroxide solution and separating the organic layer,
g) washing the organic layer of step (f) using water, saturated with water immiscible alkanol, wherein said water immiscible alkanol is selected from a group consisting of n-butanol and n-propanol or their mixture thereof, preferably n-butanol,
h) passing the washed organic layer of step (g) through acidic alumina to get a organic solution and
i) concentrating the organic solution of step (h) to obtain white amorphous powder of β-aescin having mp 220-225°C, [α]_{D} -25 - -30, pH 3.0-4.5.

Still another embodiment of the invention, the ratio of alkanol to water used in the extraction process ranges between 1:1 and 8:2

Yet another embodiment of the invention, the alkali hydroxide used for separating the organic layer is selected from a group consisting of lithium hydroxide, sodium hydroxide or potassium hydroxide, preferably sodium hydroxide.

Yet another embodiment of the invention the product *β-aescin* obtained from Indian horse chestnut *(Aesculus indica)* is in pure form.

Yet another embodiment of the invention, the yield of *β-aescin* from Indian horse chestnuts (*A. indica*) is in the range of 2.0-3.0%.

Yet another embodiment of the invention, the purity of *β-aescin* obtained from Indian horse chestnuts *(A. indica*) is in the range of 90-95 %

Yet another embodiment of the invention, the β-aescin obtained from the said process is used for commercial production of *β-aescin* for various pharmaceutical preparations.

The above said process can be used for utilization of wild growing and renewable source of Indian horse chestnut for commercial production of *β-aescin*, which otherwise go waste every year in hundred of tonnes from Himalayan region.

The present investigation provides a simplified process for purification of β-aescin from Indian horse chestnut *(Aesculus indica)*, which, comprises of (a) collection, drying and grinding of Indian horse chestnut *(Aesculus indica* seeds) (b) extraction of powdered seeds with aqueous low molecular weight alcohols (methanol/ethanol and water, 1:1-8:2) (c) fractionation of alcohol free extract with *n*-butanol or isopropanol saturated with water (d) treatment of *n*-butanol/isopropanol extract with base 0.5-1.0% sodium/patassium hydroxide and separation of aqueous and organic layer in a separating funnel (e) washing of organic layer with water saturated with butanol (f) passing the organic layer through acidic alumina (g) concentration of organic layer to yield white amorphous powder having mp 220-225°C, [α]_{D} -25 - -30, pH 3.0-4.5 and Rf. value same as β-aescin as seen on TLC (thin later chromatography coated with silica gel) plate (solvent system: chloroform, acetic acid, methanol, water; 60,32,12,6). Standard sample was procured from Sigma chemicals.

Aescin was obtained from Indian horse chestnut *(Aesculus indica* seeds) by drying, grinding and extracting with water and ethyl/methyl alcohol mixture followed by removal of organic solvent and fractionation of aqueous layer with butanol/isopropanol saturated with water. Treating the organic layer i.e. Butanol/isopropanol fraction with base in a separating funnel with gentle shaking. Washing of base treated organic layer with water saturated with butanol followed by passing of organic layer through acidic alumina. Distilling the organic layer to yield aescin as white amorphous powder.

In another embodiments of the present invention aescin was obtained in purified form having mp 220-225°C, [α]_{D} -25 - -30, pH 3.0-4.5 and Rf. value same as β-aescin as seen on TLC (thin later chromatography coated with silica gel) plate (solvent system: chloroform, acetic acid, methanol, water; 60,32,12,6).

### Brief description of the accompanying drawing

**Fig.1** shows some of the major triterpene glycosides present in aescin

The following example is given by way of illustration and therefore should not be construed to limit the scope of the present invention.

### Example

Dried and powdered Indian horse chestnut *(Aesculus indica* seeds) 1.0kg were placed in a percolator and cold extracted with an aqueous low molecular weight alcohol like methanol/ethanol in the ratio of 1:1-2:8 (V/V), 1.5-2.0 lit each time. The process of extraction was repeated for 6 to 8 times. After removal of solvent in rotavapour under reduced pressure, the extract was treated with aqueous sodium hydroxide (0.5-1.0 %) and successively fractionated with *n*-butanol or isopropanol saturated with water for 8 to 12 times. Combined organic layer which contains β-aescin was washed with aqueous sodium hydroxide and washings mixed with aqueous layer. Butanol layer was further rinsed with water saturated with butanol/isopropanol for 3-4 times. The organic layer was then passed through acidic alumina (300-400gm) in a sintered funnel under vacuum. All the separation and fractionations were carried out in a separating funnel with gentle shakings to avoid the formation of emulsion. The filtrate was concentrated in a rotavapour under vacuum to yield white, amorphous powder in 2.0-3.0% yield, having mp 220-225°C, [α]_{D} -25 - -30, pH 3.0-4.5 and Rf. value same as β-aescin as seen on TLC (thin later chromatography coated with silica gel) plate (solvent system: chloroform, acetic acid, methanol, water; 60,32,12,6) of Standard sample procured from Sigma chemicals having purity of 90-95%

### The main advantages of the present invention are:

1. *Aesculus indica* plant grows wild in temperate and sub-temperate regions of Himalayan belt from Jammu & Kashmir, Himachal Pradesh, Uttranchal and North-Eastern states hence, easily available in bulk quantity.
2. Seeds are used for the production of β-aescin which are renewable source from the plant and hence no threat to biodiversity.
3. β-aescin is a natural product and hence no threat to environment.
4. It provides a simple process for purification of β-aescin from Indian horse chestnut *(Aesculus indica* seeds)
5. It provides a easy and convenient commercial extraction process for β-aescin, a component of pharmaceutical importance from Indian horse chestnut *(Aesculus indica* seeds) growing wild in high hills of Himalaya and goes unutilized every year in hundreds of tonnes.
6. Provides an opportunity to generate employment as seed gatherers to hilly/tribal people.
7. Another usage of the present invention is the scope of commercial production of Aescin for various pharmaceutical preparations.

## Claims

1. A process for obtaining β-aescin from Indian horse chestnut *(Aesculus indica)*, said process comprising steps of:
a) extracting powdered Indian horse chestnut seeds with mixture of water and low molecular alcohol, said low molecular alcohol being selected from the group consisting of methanol and ethanol,
b) filtering the solution of step (a) to provide aqueous alkanol extract,
c) removing the alkanol from step (b) to provide essentially an aqueous extract,
d) partitioning the aqueous extract of step (c) with water immiscible lower alkanol saturated with water, wherein said water immiscible alkanol is selected from a group consisting of n-butanol and n-propanol or their mixtures thereof, preferably n-butanol,
e) separating the organic layer and aqueous layer of step (d),
f) treating the organic layer of step (e) with 0.5 to 1.0% aqueous alkali hydroxide solution and separating the organic layer,
g) washing the organic layer of step (f) using water, saturated with water immiscible alkanol, wherein said water immiscible alkanol is selected from a group consisting of n-butanol and n-propanol or their mixtures thereof, preferably n-butanol,
h) passing the washed organic layer of step (g) through acidic alumina to get a organic solution, and
i) concentrating the organic solution of step (h) to obtain white amorphous powder of β-aescin having mp 220-225°C, [α]_{D} -25 - -30, pH 3.0-4.5.

2. The process as claimed in claim 1, wherein the ratio of alkanol to water ranges between 1:1 and 8:2.

3. The process as claimed in claim 1, wherein in step (f) the alkali hydroxide is selected from a group consisting of lithium hydroxide, sodium hydroxide or potassium hydroxide, preferably sodium hydroxide.

4. The process as claimed in claim 1, wherein the above said process is a simple for the purification of β-aescin from Indian horse chestnut *(Aesculus indica)*.

5. The process as claimed in claim 1, wherein the yield of β-aescin from Indian horse chestnuts *(A. indica)* is in the range of 2.0-3.0%.

6. The process as claimed in claim 1, wherein the purity of β-aescin from Indian horse chestnuts *(A. indica)* is in the range of 90-95%.

7. The process as claimed in claim 1, wherein the above said process can be used for commercial production of β-aescin for various pharmaceutical preparations.

8. The process as claimed in claim 1, wherein above said process can be used for utilization of wild growing and renewable source of Indian horse chestnut for commercial production of β-aescin, which otherwise go waste every year in hundred of tonnes from Himalayan region.

## Patentansprüche

1. Verfahren zum Erhalt von β-Aescin aus indischer Roßkastanie (Aesculus indica), wobei das Verfahren die Schritte umfaßt:
a) Extrahieren von pulverisierten Samen der indischen Roßkastanie mit einer Mischung aus Wasser und einem Alkohol mit niedrigem Molekulargewicht, wobei der Alkohol mit niedrigem Molekulargewicht ausgewählt ist aus der Gruppe, bestehend aus Methanol und Ethanol,
b) Filtrieren der Lösung von Schritt (a), um einen wäßrigen Alkanolextrakt bereitzustellen,
c) Entfernen des Alkanols aus Schritt (b), um einen im wesentlichen wäßrigen Extrakt bereitzustellen,
d) Trennen des wäßrigen Extraktes aus Schritt (c) mit Wasser-unmischbarem niederem Alkanol, gesättigt mit Wasser, wobei der Wasser-unmischbare Alkanol ausgewählt ist aus einer Gruppe, bestehend aus n-Butanol und n-Propanol oder ihren Mischungen, bevorzugt n-Butanol,
e) Trennen der organischen Schicht und wäßrigen Schicht aus Schritt (d),
f) Behandeln der organischen Schicht aus Schritt (e) mit 0,5 bis 1,0% wäßriger Alkalihydroxid-Lösung und Trennen der organischen Schicht,
g) Waschen der organischen Schicht aus Schritt (f) unter Verwendung von Wasser, gesättigt mit Wasser-unmischbarem Alkanol, wobei der Wasser-unmischbare Alkanol ausgewählt ist aus einer Gruppe, bestehend aus n-Butanol und n-Propanol oder Mischungen davon, bevorzugt n-Butanol,
h) Passieren der gewaschenen organischen Schicht aus Schritt (g) durch saures Aluminiumoxid, um eine organische Lösung zu erhalten, und
i) Konzentrieren der organischen Lösung aus Schritt (h), um ein weißes amorphes Pulver von β-Aescin mit einem Schmelzpunkt von 220-225°C, [α]_{D}-25 - -30, pH 3,0 - 4,5, zu erhalten.

2. Verfahren nach Anspruch 1, wobei das Verhältnis von Alkanol zu Wasser im Bereich zwischen 1:1 und 8:2 liegt.

3. Verfahren nach Anspruch 1, wobei in Schritt (f) das Alkalihydroxid ausgewählt ist aus einer Gruppe, bestehend aus Lithiumhydroxid, Natriumhydroxid oder Kaliumhydroxid, bevorzugt Natriumhydroxid.

4. Verfahren nach Anspruch 1, wobei das obige Verfahren ein einfaches Verfahren zur Aufreinigung von β-Aescin aus indischer Roßkastanie (Aesculus indica) ist.

5. Verfahren nach Anspruch 1, wobei die Ausbeute von β-Aescin aus indischer Roßkastanie (A. indica) im Bereich von 2,0 - 3,0% liegt.

6. Verfahren nach Anspruch 1, wobei die Aufreinigung von β-Aescin aus indischer Roßkastanie (A. indica) im Bereich von 90-95% liegt.

7. Verfahren nach Anspruch 1, wobei das obige Verfahren zur kommerziellen Herstellung von β-Aescin für verschiedenen pharmazeutische Zubereitungen verwendet werden kann.

8. Verfahren nach Anspruch 1, wobei das obige Verfahren zur Verwendung von wildwachsenden und emeuerbaren Quellen von indischer Roßkastanie zur kommerziellen Herstellung von β-Aescin verwendet werden kann, welche ansonsten jährlich in Hunderten von Tonnen aus der Himalaya-Region verschwendet werden.

## Revendications

1. Procédé d'obtention de β-aescéine à partir du marronnier de l'Himalaya *(Aesculus indica*), ledit procédé comprenant les étapes consistant à :
a) extraire des graines de marronnier de l'Himalaya réduites en poudre avec un mélange d'eau et d'alcool de faible masse moléculaire, ledit alcool de faible masse moléculaire étant choisi dans le groupe consistant en le méthanol et l'éthanol,
b) filtrer la solution de l'étape (a) pour donner un extrait d'alkanol aqueux,
c) éliminer l'alkanol de l'étape (b) pour donner essentiellement un extrait aqueux,
d) partager l'extrait aqueux de l'étape (c) avec un alkanol inférieur non miscible dans l'eau saturé d'eau, dans lequel ledit alkanol non miscible dans l'eau est choisi dans un groupe consistant en le n-butanol et le n-propanol ou leurs mélanges de ceux-ci, préférablement le n-butanol,
e) séparer la couche organique et la couche aqueuse de l'étape (d),
f) traiter la couche organique de l'étape (e) avec une solution aqueuse d'un hydroxyde alcalin à 0,5 à 1,0% et séparer la couche organique,
g) laver la couche organique de l'étape (f) en utilisant de l'eau, un alkanol non miscible dans l'eau saturé d'eau, dans lequel ledit alkanol non miscible dans l'eau est choisi dans un groupe consistant en le n-butanol et le n-propanol ou leurs mélanges de ceux-ci, préférablement le n-butanol,
h) passer la couche organique lavée de l'étape (g) à travers de l'alumine acide pour obtenir une solution organique, et
i) concentrer la solution organique de l'étape (h) pour obtenir une poudre amorphe blanche de β-aescéine ayant un point de fusion de 220 à 225°C, [α]_{D}-25 - - 30, pH 3,0 à 4,5.

2. Procédé selon la revendication 1, dans lequel le rapport de l'alkanol à l'eau est dans la gamme de 1:1 à 8:2.

3. Procédé selon la revendication 1, dans lequel à l'étape (f) l'hydroxyde alcalin est choisi dans un groupe consistant en l'hydroxyde de lithium, l'hydroxyde de sodium ou l'hydroxyde de potassium, préférablement l'hydroxyde de sodium.

4. Procédé selon la revendication 1, dans lequel ledit procédé ci-dessus est un procédé simple pour la purification de la β-aescéine obtenue à partir du marronnier de l'Himalaya (*Aesculus indica*).

5. Procédé selon la revendication 1, dans lequel le rendement de la β-aescéine obtenue à partir du marronnier de l'Himalaya (*A. indica*) est dans la gamme de 2,0 à 3,0%.

6. Procédé selon la revendication 1, dans lequel la pureté de la β-aescéine obtenue à partir du marronnier de l'Himalaya (*A. indica*) est dans la gamme de 90 à 95%.

7. Procédé selon la revendication 1, dans lequel ledit procédé ci-dessus peut être utilisé pour la production commerciale de β-aescéine pour diverses préparations pharmaceutiques.

8. Procédé selon la revendication 1, dans lequel ledit procédé ci-dessus peut être utilisé pour une utilisation d'une source sauvage et renouvelable de marronnier de l'Himalaya pour la production commerciale de β-aescéine, qui sinon part en déchet tous les ans par centaine de tonnes de la région himalayenne.
